# EUROPEAN PATENT APPLICATION

(11) **EP 2 382 873 A1**
(43) Date of publication of application: **02.11.2011**
(21) Application number: 10182363.1
(22) Date of filing: 13.07.2006
(51) Int. Cl.: A23L 1/30, A61K 36/00, A61K 31/07, A61K 31/01, A61K 31/045, A61K 8/34, A61K 8/31, A61K 31/122, A61P 17/16

(54) **Novel use of organic compound**

(30) Priority: 15.07.2005 EP 05015426; 21.04.2006 EP 06008285
(62) Divisional of application: 06776214.6
(71) Applicant: DSM IP Assets B.V., 6411 TE Heerlen (NL)
(72) Inventor: Goralczyk, Regina, 79639, Grenzach-Wyhlen (DE); Wertz, Karin, 79618, Rheinfelden (DE)
(74) Representative: Kurt, Manfred

(57) **Abstract**

The present invention is directed to the use of a compound selected from the group consisting of β-carotene, lutein, lycopene and β-cryptoxanthin, and mixtures thereof with CoQ-I O for maintaining the energy metabolism, the energy flow and/or the energy production in skin or of skin of animals including humans, for maintaining the respiratory function of the skin of animals including humans, for energizing the skin, maintaining and supporting the radiance and natural glow of the skin of animals including humans and for promoting a healthy appearance of the skin of animals including humans for preventing UV-A radiation-inducted mtDNA mutagenesis in skin of animals including humans, as well as for the manufacture of a composition, preferably an orally applicable composition, for these uses and the corresponding methods. The present invention is further directed to the use of a compound selected from the group consisting of β-carotene, lutein, lycopene, and β-cryptoxanthin, and mixtures thereof and combinations thereof with CoQ-10 in sunscreens as well as daily care products to improve their photoprotective potential, as well as to their use as effective micronutrients for skin maintenance. Furthermore the present invention is directed to compositions, preferably orally applicable compositions, comprising a certain amount of at least one compound selected from the group consisting of β-carotene, lutein, lycopene and β-cryptoxanthin and mixtures thereof and combinations thereof with CoQ-10 as active ingredient, characterized in that the amount is effective for such uses as mentioned above.

## Description

The present invention is directed to the use of a compound selected from the group consisting of β-carotene, lutein, lycopene and β-cryptoxanthin and mixtures thereof and combinations thereof with coenzyme Q10 (CoQ-10), including all their E- and Z-stereoisomers, for maintaining the energy metabolism, the energy flow and/or the energy production in skin or of skin of animals including humans, for maintaining the respiratory function of the skin of animals including humans, for energizing the skin, maintaining and supporting the radiance and natural glow of the skin of animals including humans and for promoting a healthy appearance of the skin of animals including humans, for preventing UV-A radiation-induced mtDNA mutagenesis in skin of animals including humans, as well as to the use of such carotenoids for the manufacture of a composition, preferably an orally applicable composition, for these uses and to the corresponding methods. The present invention is further directed to the use of a compound selected from the group consisting of β-carotene, lutein, lycopene and β-cryptoxanthin and mixtures thereof and combinations thereof with CoQ-10 in sunscreens as well as daily care products to improve their photoprotective potential, as well as to their use as effective micronutrients for skin maintenance. Furthermore the present invention is directed to compositions, preferably orally applicable compositions, comprising a certain amount of at least one compound selected from the group consisting of β-carotene, lutein, lycopene and β-cryptoxanthin and mixtures thereof and combinations thereof with CoQ-10 and their stereoisomers. In the present description and claims the designations "lutein" and "β-cryptoxanthin" include mono- and di-fatty acid esters (preferably esters with saturated alkanoic acids such as acetic, propionic, laurinic, myristinic, palmitic, stearic and succinic acid, mono- unsaturated fatty acids (PUFAs) such as oleic acid, and poly-unsaturated fatty acids such as linolic, linoleic, docosahexaenoic and arachidonic acid) thereof.

UV-A radiation, i.e. radiation with a light of wavelengths of 320 to 400 nm exerts direct effects on dermal fibroblasts. In particular, chronic repetitive exposure to UV-A radiation leads to the generation of mitochondrial (mt) DNA mutations in these cells, which is 40% higher than in non-exposed skin (Berneburg al., Journal of Investigative Dermatology, 2004). Such a mutation is the common deletion in mitochondrial DNA (mtDNA) of a specific 4977-bp fragment. The 4977-bp deletion affects inter alia genes encoding 7 polypeptide components of the mitochondrial respiratory chain. This mt deletion is detectable in skin even after cessation of irradiation, and can be detected months after, accumulating further at levels up to 32fold higher. UV-A radiation thus poses a permanent chronic risk to skin, even when not exposed, i.e. also thereafter. The mitochondrial DNA damage leads to a deterioriation of the function of the mitochondrial respiratory chain, consequently followed by an impaired energy metabolism of the acutely and previously exposed skin compared with the energy metabolism of never-UV exposed skin. The bioenergetic deficit and declined respiration leads to cellular dysfunction. Thus, a continuous accumulation of mt DNA deletions needs to be prevented on a permanent basis, i.e. even when an individual is not UV exposed, to avoid the detrimental consequences on the energy metabolism of the skin cells.

It has now been found that a compound selected from the group consisting of β-carotene, lutein, lycopene and β-cryptoxanthin and mixtures thereof and combinations thereof with CoQ-10, especially a carotenoid selected from the group consisting of lutein, lycopene and β-cryptoxanthin, and mixtures thereof may prevent such damage in form of mutations/deletions in the mtDNA thus leading to the maintenance of the energy metabolism of the skin of animals including humans, to the maintenance of the respiratory function of the skin of animals including humans to the maintenance and support of the radiance and natural glow as well a to the promotion of a healthy appearance of the skin of animals including man. The respiratory function of the skin predominantly encompasses the oxidative phosphorylation taking place in the skin cells which is a precondition for the production of adenosine triphosphate (ATP) from nutrients.

Thus, an object of the present invention is the use of a compound selected from the group consisting of β-carotene, lutein, lycopene and β-cryptoxanthin and mixtures thereof and combinations thereof with CoQ-10 for maintaining the energy metabolism, the energy flow and/or the energy production in skin or of skin of animals including humans.

Another object of the present invention is the use of a compound selected from the group consisting of β-carotene, lutein, lycopene and β-cryptoxanthin, and mixtures thereof and combinations thereof with CoQ-10 for maintaining the respiratory function of the skin of animals including humans.

Another object of the present invention is the use of a compound selected from the group consisting of β-carotene, lutein, lycopene and β-cryptoxanthin, and mixtures thereof and combinations thereof with CoQ10, for energizing the skin, maintaining and supporting the radiance and natural glow of the skin of animals including humans and for promoting a healthy appearance of the skin of animals including humans.

A further object of the present invention is the use of a compound selected from the group consisting of β-carotene, lutein, lycopene and β-cryptoxanthin and mixtures thereof and combinations thereof with CoQ-10 for preventing UV-A radiation-induced mtDNA mutagenesis in skin of animals including humans.

In specific embodiments the present invention relates to the uses defined above of a carotenoid selected from the group consisting of lutein, lycopene, β-cryptoxanthin, and mixtures thereof.

Special embodiments of the present invention are
◇ the use of β-carotene for maintaining the energy metabolism, the energy flow and/or the energy production in skin or of skin of animals including humans;
◇ the use of β-cryptoxanthin for maintaining the energy metabolism, the energy flow and/or the energy production in skin or of skin of animals including humans;
◇ the use of lutein for maintaining the energy metabolism, the energy flow and/or the energy production in skin or of skin of animals including humans;
0 the use of lycopene for maintaining the energy metabolism, the energy flow and/or the energy production in skin or of skin of animals including humans;
0 the use of a combination of lutein and lycopene for maintaining the energy metabolism, the energy flow and/or the energy production in skin or of skin of animals including humans;
◇ the use of a combination of lutein and β-cryptoxanthin for maintaining the energy metabolism, the energy flow and/or the energy production in skin or of skin of animals including humans;
0 the use of a combination of lutein and CoQ-10 for maintaining the energy metabolism, the energy flow and/or the energy production in skin or of skin of animals including humans;
◇ the use of a combination of lycopene and β-cryptoxanthin for maintaining the energy metabolism, the energy flow and/or the energy production in skin or of skin of animals including humans;
0 the use of a combination of lycopene and CoQ-10 for maintaining the energy metabolism, the energy flow and/or the energy production in skin or of skin of animals including humans;
◇ the use of a combination of CoQ-10 and β-cryptoxanthin for maintaining the energy metabolism, the energy flow and/or the energy production in skin or of skin of animals including humans;
◇ the use of a combination of lycopene, lutein and β-cryptoxanthin for maintaining the energy metabolism, the energy flow and/or the energy production in skin or of skin of animals including humans;
◇ the use of a combination of lycopene, CoQ10 and β-cryptoxanthin for maintaining the energy metabolism, the energy flow and/or the energy production in skin or of skin of animals including humans;
◇ the use of a combination of CoQ10, lutein and β-cryptoxanthin for maintaining the energy metabolism, the energy flow and/or the energy production in skin or of skin of animals including humans;
◇ the use of a combination of CoQ10, lutein and lycopene for maintaining the energy metabolism, the energy flow and/or the energy production in skin or of skin of animals including humans;
◇ the use of a combination of β-cryptoxanthin, lutein and lycopene for maintaining the energy metabolism, the energy flow and/or the energy production in skin or of skin of animals including humans;
◇ the use of a combination of β-cryptoxanthin, lutein, CoQ-10 and lycopene for maintaining the energy metabolism, the energy flow and/or the energy production in skin or of skin of animals including humans;
◇ the use of a combination of β-carotene and lycopene for maintaining the energy metabolism, the energy flow and/or the energy production in skin or of skin of animals including humans;
◇ the use of a combination of β-carotene and lutein for maintaining the energy metabolism, the energy flow and/or the energy production in skin or of skin of animals including humans;
◇ the use of a combination of β-carotene and CoQ-10 for maintaining the energy metabolism, the energy flow and/or the energy production in skin or of skin of animals including humans;
◇ the use of a combination of β-carotene and β-cryptoxanthin, for maintaining the energy metabolism, the energy flow and/or the energy production in skin or of skin of animals including humans;
◇ the use of a combination of β-carotene, β-cryptoxanthin, and lutein for maintaining the energy metabolism, the energy flow and/or the energy production in skin or of skin of animals including humans;
◇ the use of a combination of β-carotene, β-cryptoxanthin and CoQ-10 for maintaining the energy metabolism, the energy flow and/or the energy production in skin or of skin of animals including humans;
◇ the use of a combination of β-carotene, β-cryptoxanthin and lycopene for maintaining the energy metabolism, the energy flow and/or the energy production in skin or of skin of animals including humans;
◇ the use of a combination of β-carotene, lutein and CoQ-10 for maintaining the energy metabolism, the energy flow and/or the energy production in skin or of skin of animals including humans;
◇ the use of a combination of β-carotene, lutein and lycopene for maintaining the energy metabolism, the energy flow and/or the energy production in skin or of skin of animals including humans;
◇ the use of a combination of β-carotene, CoQ-10 and lycopene for maintaining the energy metabolism, the energy flow and/or the energy production in skin or of skin of animals including humans;
◇ the use of a combination of β-carotene, CoQ-10 and lutein for maintaining the energy metabolism, the energy flow and/or the energy production in skin or of skin of animals including humans;
◇ the use of a combination of β-carotene, lutein, CoQ-10 and lycopene for maintaining the energy metabolism, the energy flow and/or the energy production in skin or of skin of animals including humans;
◇ the use of a combination of β-carotene, β-cryptoxanthin, CoQ-10 and lycopene for maintaining the energy metabolism, the energy flow and/or the energy production in skin or of skin of animals including humans;
◇ the use of a combination of β-carotene, β-cryptoxanthin, lutein and lycopene for maintaining the energy metabolism, the energy flow and/or the energy production in skin or of skin of animals including humans;
◇ the use of a combination of β-carotene, β-cryptoxanthin, lutein, and CoQ-10 for maintaining the energy metabolism, the energy flow and/or the energy production in skin or of skin of animals including humans;
◇ the use of a combination of β-carotene, β-cryptoxanthin, lutein, CoQ-10 and lycopene for maintaining the energy metabolism, the energy flow and/or the energy production in skin or of skin of animals including humans;
◇ the use of β-carotene for maintaining the respiratory function of the skin of animals including humans;
◇ the use of β-cryptoxanthin for maintaining the respiratory function of the skin of animals including humans;
◇ the use of lutein for maintaining the respiratory function of the skin of animals including humans;
◇ the use of lycopene for maintaining the respiratory function of the skin of animals including humans;
◇ the use of a combination of lutein and lycopene for maintaining the respiratory function of the skin of animals including humans;
◇ the use of a combination of lutein and β-cryptoxanthin for maintaining the respiratory function of the skin of animals including humans;
◇ the use of a combination of lutein and CoQ-10 for maintaining the respiratory function of the skin of animals including humans;
◇ the use of a combination of lycopene and β-cryptoxanthin for maintaining the respiratory function of the skin of animals including humans;
◇ the use of a combination of lycopene and CoQ-10 for maintaining the respiratory function of the skin of animals including humans;
◇ the use of a combination of CoQ-10 and β-cryptoxanthin for maintaining the respiratory function of the skin of animals including humans;
◇ the use of a combination of lycopene, lutein and β-cryptoxanthin for maintaining the respiratory function of the skin of animals including humans;
◇ the use of a combination of lycopene, CoQ10 and β-cryptoxanthin for maintaining the respiratory function of the skin of animals including humans;
◇ the use of a combination of CoQ10, lutein and β-cryptoxanthin for maintaining the respiratory function of the skin of animals including humans;
◇ the use of a combination of CoQ10, lutein and lycopene for maintaining the respiratory function of the skin of animals including humans;
◇ the use of a combination of β-cryptoxanthin, lutein and lycopene for maintaining the respiratory function of the skin of animals including humans;
◇ the use of a combination of β-cryptoxanthin, lutein, CoQ-10 and lycopene for maintaining the respiratory function of the skin of animals including humans;
◇ the use of a combination of β-carotene, β-cryptoxanthin, and lutein for maintaining the respiratory function of the skin of animals including humans;
◇ the use of a combination of β-carotene, β-cryptoxanthin and CoQ-10 for maintaining the respiratory function of the skin of animals including humans;
◇ the use of a combination of β-carotene, β-cryptoxanthin and lycopene for maintaining the respiratory function of the skin of animals including humans;
◇ the use of a combination of β-carotene, lutein and CoQ-10 for maintaining the respiratory function of the skin of animals including humans;
◇ the use of a combination of β-carotene, lutein and lycopene for maintaining the respiratory function of the skin of animals including humans;
◇ the use of a combination of β-carotene, CoQ-10 and lycopene for maintaining the respiratory function of the skin of animals including humans;
◇ the use of a combination of β-carotene, CoQ-10 and lutein for maintaining the respiratory function of the skin of animals including humans;
◇ the use of a combination of β-carotene and lycopene for maintaining the respiratory function of the skin of animals including humans;
◇ the use of a combination of β-carotene and lutein for maintaining the respiratory function of the skin of animals including humans;
◇ the use of a combination of β-carotene and CoQ-10 for maintaining the respiratory function of the skin of animals including humans;
◇ the use of a combination of β-carotene and β-cryptoxanthin for maintaining the respiratory function of the skin of animals including humans;
◇ the use of a combination of β-carotene, lutein, CoQ-10 and lycopene for maintaining the respiratory function of the skin of animals including humans;
◇ the use of a combination of β-carotene, β-cryptoxanthin, CoQ-10 and lycopene for maintaining the respiratory function of the skin of animals including humans;
◇ the use of a combination of β-carotene, β-cryptoxanthin, lutein and lycopene for maintaining the respiratory function of the skin of animals including humans;
◇ the use of a combination of β-carotene, β-cryptoxanthin, lutein, and CoQ-10 for maintaining the respiratory function of the skin of animals including humans;
◇ the use of a combination of β-carotene, β-cryptoxanthin, lutein, CoQ-10 and lycopene for maintaining the respiratory function of the skin of animals including humans;

◇ the use of β-carotene for preventing UV-A radiation-induced mtDNA mutagenesis in skin of animals including humans;
◇ the use of β-cryptoxanthin for preventing UV-A radiation-induced mtDNA mutagenesis in skin of animals including humans;
◇ the use of lutein for preventing UV-A radiation-induced mtDNA mutagenesis in skin of animals including humans;
◇ the use of lycopene for preventing UV-A radiation-induced mtDNA mutagenesis in skin of animals including humans;
◇ the use of a combination of lutein and lycopene for preventing UV-A radiation-induced mtDNA mutagenesis in skin of animals including humans;
◇ the use of a combination of lutein and β-cryptoxanthin for preventing UV-A radiation-induced mtDNA mutagenesis in skin of animals including humans;
◇ the use of a combination of lutein and CoQ-10 for preventing UV-A radiation-induced mtDNA mutagenesis in skin of animals including humans;
◇ the use of a combination of lycopene and β-cryptoxanthin for preventing UV-A radiation-induced mtDNA mutagenesis in skin of animals including humans;
◇ the use of a combination of lycopene and CoQ-10 f= for preventing UV-A radiation-induced mtDNA mutagenesis in skin of animals including humans;
◇ the use of a combination of CoQ-10 and β-cryptoxanthin for preventing UV-A radiation-induced mtDNA mutagenesis in skin of animals including humans;
◇ the use of a combination of lycopene, lutein and β-cryptoxanthin for preventing UV-A radiation-induced mtDNA mutagenesis in skin of animals including humans;
◇ the use of a combination of lycopene, CoQ10 and β-cryptoxanthin for preventing UV-A radiation-induced mtDNA mutagenesis in skin of animals including humans;
◇ the use of a combination of CoQ10, lutein and β-cryptoxanthin for preventing UV-A radiation-induced mtDNA mutagenesis in skin of animals including humans;
◇ the use of a combination of CoQ 10, lutein and lycopene for preventing UV-A radiation-induced mtDNA mutagenesis in skin of animals including humans;
◇ the use of a combination of β-cryptoxanthin, lutein and lycopene for preventing UV-A radiation-induced mtDNA mutagenesis in skin of animals including humans;
◇ the use of a combination of β-cryptoxanthin, lutein, CoQ-10 and lycopene for preventing UV-A radiation-induced mtDNA mutagenesis in skin of animals including humans;
◇ the use of a combination of β-carotene and lycopene for preventing UV-A radiation-induced mtDNA mutagenesis in skin of animals including humans;
◇ the use of a combination of β-carotene and lutein for preventing UV-A radiation-induced mtDNA mutagenesis in skin of animals including humans;
◇ the use of a combination of β-carotene and CoQ-10 for preventing UV-A radiation-induced mtDNA mutagenesis in skin of animals including humans;
◇ the use of a combination of β-carotene and β-cryptoxanthin for preventing UV-A radiation-induced mtDNA mutagenesis in skin of animals including humans;
◇ the use of a combination of β-carotene, β-cryptoxanthin, and lutein for preventing UV-A radiation-induced mtDNA mutagenesis in skin of animals including humans;
◇ the use of a combination of β-carotene, β-cryptoxanthin and CoQ-10 for preventing UV-A radiation-induced mtDNA mutagenesis in skin of animals including humans;
◇ the use of a combination of β-carotene, β-cryptoxanthin and lycopene for preventing UV-A radiation-induced mtDNA mutagenesis in skin of animals including humans;
◇ the use of a combination of β-carotene, lutein and CoQ-10 for preventing UV-A radiation-induced mtDNA mutagenesis in skin of animals including humans;
◇ the use of a combination of β-carotene, lutein and lycopene for preventing UV-A radiation-induced mtDNA mutagenesis in skin of animals including humans;
◇ the use of a combination of β-carotene, CoQ-10 and lycopene for preventing UV-A radiation-induced mtDNA mutagenesis in skin of animals including humans;
◇ the use of a combination of β-carotene, CoQ-10 and lutein for preventing UV-A radiation-induced mtDNA mutagenesis in skin of animals including humans;
◇ the use of a combination of β-carotene, lutein, CoQ-10 and lycopene for preventing UV-A radiation-induced mtDNA mutagenesis in skin of animals including humans;
◇ the use of a combination of β-carotene, β-cryptoxanthin, CoQ-10 and lycopene for preventing UV-A radiation-induced mtDNA mutagenesis in skin of animals including humans;
◇ the use of a combination of β-carotene, β-cryptoxanthin, lutein and lycopene for preventing UV-A radiation-induced mtDNA mutagenesis in skin of animals including humans;
◇ the use of a combination of β-carotene, β-cryptoxanthin, lutein, and CoQ-10 for preventing UV-A radiation-induced mtDNA mutagenesis in skin of animals including humans;
◇ the use of a combination of β-carotene, β-cryptoxanthin, lutein, CoQ-10 and lycopene for preventing UV-A radiation-induced mtDNA mutagenesis in skin of animals including humans;
whereby in those special embodiments of the present invention concerning the uses cited above the named compounds/combinations are preferably the only active ingredients for maintaining the energy metabolism, the energy flow and/or the energy production in skin or of skin of animals including humans, for maintaining the respiratory function of the skin of animals including humans, for energizing the skin, maintaining and supporting the radiance and natural glow of the skin of animals including humans and for promoting a healthy appearance of the skin of animals including humans and/or for preventing UV-A radiation-induced mtDNA mutagenesis in skin of animals including humans in compositions used for these purposes.

In further embodiments of the present invention concerning the uses cited above the named compounds/combinations are preferably further combined with at least one compound selected from the group consisting of β-carotene, vitamin C, vitamin E, resveratrol and (-)-epigallocatechin gallate, more preferably with at least one compound selected from the group consisting of vitamin C, vitamin E, resveratrol and (-)-epigallocatechin gallate, most preferably with resveratrol and/or (-)-epigallocatechin gallate.

The daily dosage for humans (usually determined for a 70 kg person) for lutein should be more than 0.1 mg, preferably more than 0.5 mg, for lycopene more than 0.1 mg, preferably more than 1.0 mg, for β-cryptoxanthin more than 0.1 mg, preferably more than 0.5 mg, for β-carotene more than 0.1 mg, preferably more than 0.3 mg, and/or for CoQ-10 more than 1 mg, preferably more than 10 mg (most preferably 30-60 mg).

For usual applications the daily dosage for humans (usually determined for a 70 kg person) for lutein should not exceed 40 mg, preferably not exceed 25 mg, for lycopene not exceed 60 mg, preferably not exceed 30 mg, for β-cryptoxanthin not exceed 20 mg, preferably not exceed 15 mg, for β-carotene not exceed 20 mg, preferably not exceed 10 mg, and/or for CoQ-10 not exceed 200 mg, preferably not exceed 60 mg.

In some embodiments of the invention the daily dosage for humans (70 kg person) for lutein can be between 0.1 to 40 mg, more preferably between 0.5 to 25 mg, for lycopene between 0.1 to 60 mg, more preferably between 1.0 to 30 mg, for β-cryptoxanthin between 0.1 to 20 mg, more preferably between 0.5 to 15 mg, for β-carotene between 0.1 to 20 mg, more preferably between 0.3 to 10 mg, and/or for CoQ-10 between 1 to 200 mg, more preferably between 10 to 60 mg.

For humans (70 kg person) the daily dosage preferably may vary for vitamin C between 100 mg and 5 g, more preferably between 200 mg and 1.5 g, for vitamin E between 15 mg and 2 g, more preferably between 15 and 500 mg, for resveratrol between 1 and 100 mg, more preferably between 5 and 50 mg, and/or for (-)-epigallocatechin gallate between 10 mg and 1.5 g, more preferably between 50 and 300 mg.

Also an object of the present invention is the use of a compound selected from the group consisting of β-carotene, lutein, lycopene and β-cryptoxanthin, and mixtures thereof and combinations thereof with CoQ-10 as well as their stereoisomers, and/or in the case of lutein and β-cryptoxanthin their mono-and di-esters, and mixtures thereof, especially the use of a carotenoid selected from the group consisting of lutein, lycopene, β-cryptoxanthin, including all their stereoisomers, and/or in the case of lutein and β-cryptoxanthin their mono- and di- fatty acid esters, and mixtures thereof, for the manufacture of a composition, preferably an orally applicable composition, used for maintaining the energy metabolism of/the energy flow in/the energy production in skin of animals including humans, for maintaining the respiratory function of the skin of animals including humans, and for preventing UV-A radiation-induced mtDNA mutagenesis in skin of animals including humans.

A further object of the present invention is a composition, preferably an orally applicable composition, comprising an amount of at least one compound selected from the group consisting of β-carotene, lutein, lycopene and β-cryptoxanthin and mixtures thereof and combinations thereof with CoQ-10, preferably from the group consisting of lutein, lycopene and β-cryptoxanthin, including all their stereoisomers, as active ingredient(s), characterized in that the amount is effective for maintaining the energy metabolism of/the energy flow in/the energy production in skin of animals including humans, for maintaining the respiratory function of the skin of animals including humans for maintaining and supporting the radiance and natural glow of the skin of animals including humans and for promoting a healthy appearance of the skin of animals including humans, and for preventing UV-A radiation-induced mtDNA mutagenesis in skin of animals including humans.

A further object of the present invention is a composition, preferably an orally applicable composition, comprising a certain amount of at least one carotenoid selected from the group consisting of lutein, lycopene, β-cryptoxanthin and their stereoisomers, and/or in the case of lutein and β-cryptoxanthin their mono-and di-esters, as active ingredient, characterized in that the amount is effective for maintaining the energy metabolism of/the energy flow in/the energy production in skin of animals including humans, for maintaining the respiratory function of the skin of animals including humans, for maintaining and supporting the radiance and natural glow of the skin of animals including man and for promoting a healthy appearance of the skin of animals including man and for preventing UV-A radiation-induced mtDNA mutagenesis in skin of animals including humans.

The compositions according to the present invention encompass topically applicable and orally applicable compositions. Preferred are orally applicable compositions.

However, excluded from the scope of the present invention are topical or oral compositions comprising β-carotene in admixture with lycopene in a weight ratio of from lower than 1, particularly 0,95 : 1, to 1 : 50 and optionally containing in addition lutein and/or cryptoxanthin.

The term "topically applicable composition" comprises any type of "cosmetic preparation" or "cosmetic composition" being suitable for applying onto the skin of animals such as liquid or solid oil-in-water emulsions, water-in-oil emulsions, multiple emulsions, microemulsions, PET-emulsions, bickering emulsions, hydrogels, alcoholic gels, lipogels, one or multiphase solutions, foams, ointments, plasters, suspensions, powders, crèmes, cleanser, soaps and other usual compositions, which can also be applied by pens, as masks or as prays.

The term "cosmetic preparation" or "cosmetic composition" as used in the present application refers to cosmetic compositions as defined under the heading "Kosmetika" in Römpp Lexikon Chemie, 10th edition 1997, Georg Thieme Verlag Stuttgart, New York.

The compositions of the invention can also contain usual cosmetic or pharmaceutical adjuvants and additives, such as preservatives/ antioxidants, fatty substances/ oils, water, organic solvents, silicones, thickeners, softeners, emulsifiers, sunscreens, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, e.g. those suited for providing a photoprotective effect by physically blocking out ultraviolet radiation, or any other ingredients usually formulated into cosmetics or medicaments.

An additional amount of antioxidants/ preservatives is generally preferred. Based on the invention all known antioxidants usually formulated into cosmetics or medicaments can be used. Especially preferred are antioxidants chosen from the group consisting of amino acids (e.g. glycine, histidine, tyrosine, tryptophane) and their derivatives, imidazole (e.g. urocanic acid) and derivatives, peptides such as D,L-carnosine, D-carnosine, L-carnosine and derivatives (e.g. anserine), carotenoids other than β-carotene, lutein, lycopene and β-cryptoxanthin, chlorogenic acid and derivatives, lipoic acid and derivatives (e.g. dihydrolipoic acid), aurothioglucose, propylthiouracil and other thiols (e.g. thioredoxine, glutathione, cysteine, cystine, cystamine and its glycosyl-, N-acetyl-, methyl-, ethyl-, propyl-, amyl-, butyl- and lauryl-, palmitoyl-, oleyl-, y-linoleyl-, cholesteryl- and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinsulfoximine, buthioninsulfone, penta-, hexa-, heptathioninsulfoximine) in very low compatible doses (e.g. pmol to µmol/kg), additionally (metal)-chelators (such as α-hydroxyfatty acids, palmic-, phytinic acid, lactoferrin), α-hydroxyacids (such as citric acid, lactic acid, malic acid), huminic acid, gallic acid, gallic extracts, bilirubin, biliverdin, EDTA (ethylene diamine tetraacetate), EGTA and its derivatives, unsaturated fatty acids and their derivatives (such as γ-linoleic acid, linolic acid, oleic acid), folic acid and its derivatives, vitamin C and derivatives thereof (such as ascorbylpalmitate and ascorbyltetraisopalmitate, Mg-ascorbylphosphate, Na-ascorbylphosphate, Na-ascorbylacetate), tocopherol and derivatives (such as vitamin-E-acetate), mixtures of nat. vitamin E, vitamin A and derivatives (vitamin-A-palmitate and -acetate) as well as coniferylbenzoat, rutinic acid and derivatives, α-glycosylrutin, ferulic acid, furfurylidenglucitol, carnosine, butylhydroxytoluene, butylhydroxyanisole, trihydroxybutyrophenone, urea and its derivatives, mannose and derivatives, zinc and derivatives (e.g. ZnO, ZnSO₄), selenium and derivatives (e.g. selenomethionin), stilbenes and derivatives (such as stilbenoxide, trans-stilbenoxide) and suitable derivatives (salts, esters, ethers, sugars, nucleotides, nucleosides, peptides and lipids) of the named active ingredients. One or more preservatives/antioxidants may be present in an amount about 0.01 wt.% to about 10 wt.% of the total weight of the composition of the present invention. Preferably, one or more preservatives/antioxidants are present in an amount about 0.1 wt.% to about 1 wt.%.

Typically, topical compositions also contain surface active ingredients like emulsifiers, solubilizers and the like. An emulsifier enables two or more immiscible components to be combined homogeneously. Moreover, the emulsifier can act to stabilize the composition. Emulsifiers that may be used in the present invention in order to form O/W, W/O, O/W/O or W/O/W emulsions/ microemulsions include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polyglyceryl-4 oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further suitable emulsifiers are phosphate esters and the salts thereof such as cetyl phosphate (Amphisol^{®} A), diethanolamine cetyl phosphate (Amphisol^{®}), potassium cetyl phosphate (Amphisol^{®} K), sodium glyceryl oleate phosphate, hydrogenated vegetable glyceride phosphates and mixtures thereof. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof. The preferred emulsifiers are cetyl phosphate (Amphisol^{®} A), diethanolamine cetyl phosphate (Amphisol^{®}), potassium cetyl phosphate (Amphisol^{®} K), PVP Eicosene copolymer, acrylates/C₁₀₋₃₀-alkyl acrylate crosspolymer, PEG-20 sorbitan isostearate, sorbitan isostearate, and mixtures thereof. The one or more emulsifiers are present in a total amount about 0.01 wt.% to about 20 wt.% of the total weight of the composition of the present invention. Preferably, about 0.1 wt.% to about 10 wt.% of emulsifiers is used.

The lipid phase of the topical compositions can advantageously be chosen from:
◇ mineral oils and mineral waxes;
◇ oils such as triglycerides of caprinic acid or caprylic acid and castor oil;
◇ oils or waxes and other natural or synthetic oils, in a preferred embodiment esters of fatty acids with alcohols e.g. isopropanol, propylene glycol, glycerin or esters of fatty alcohols with carboxylic acids or fatty acids;
◇ alkylbenzoates; and/or
◇ silicone oils such as dimethylpolysiloxane, diethylpolysiloxane, diphenylpolysiloxane, cyclomethicones and mixtures thereof.

Exemplary fatty substances which can be incorporated in the oil phase of the emulsion, microemulsion, oleo gel, hydrodispersion or lipodispersion of the present invention are advantageously chosen from esters of saturated and/or unsaturated, linear or branched alkyl carboxylic acids with 3 to 30 carbon atoms, and saturated and/or unsaturated, linear and/or branched alcohols with 3 to 30 carbon atoms as well as esters of aromatic carboxylic acids and of saturated and/or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexyllaureate, n-decyloleat, isooctylstearate, isononylstearate, isononylisononanoate, 2- ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as synthetic, half-synthetic or natural mixtures of such esters e.g. jojoba oil.

Other fatty components suitable for use in the topical compositions of the present invention include polar oils such as lecithines and fatty acid triglycerides, namely triglycerol esters of saturated and/or unsaturated, straight or branched carboxylic acid with 8 to 24 carbon atoms, preferably of 12 to 18 carbon-atoms whereas the fatty acid triglycerides are preferably chosen from synthetic, half synthetic or natural oils (e.g. cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalane and squalene, polyolefines, hydrogenated polyisobutenes and isohexadecanes, favored polyolefines are polydecenes; dialkyl ethers such as dicaprylylether; linear or cyclic silicone oils such as preferably cyclomethicones (octamethylcyclotetrasiloxane; cetyldimethicone, hexamethylcyclotrisiloxane, polydimethylsiloxane, poly(methylphenylsiloxane) and mixtures thereof.

Other fatty components which can advantageously be incorporated in topical compositions of the present invention are isoeikosane; neopentylglykoldiheptanoate; propylenglykoldicaprylate/ dicaprate; caprylic/ capric/ diglycerylsuccinate; butylenglykol caprylat/caprat; C₁₂₋₁₃-alkyllactate; di-C₁₂₋₁₃ alkyltartrate; triisostearin; dipentaerythrityl hexacaprylat/hexacaprate; propylenglycolmonoisostearate; tricaprylin; dimethylisosorbid. Especially beneficial is the use of mixtures C₁₂₋₁₅-alkylbenzoate and 2-ethylhexylisostearate, mixtures C₁₂₋₁₅-alkylbenzoate and isotridecylisononanoate as well as mixtures of C₁₂₋₁₅-alkylbenzoate, 2-ethylhexylisostearate and isotridecylisononanoate.

The oily phase of the compositions of the present invention can also contain natural vegetable or animal waxes such as bee wax, china wax, bumblebee wax and other waxes of insects as well as shea butter and cocoa butter.

A moisturizing agent may be incorporated into a topical composition of the present invention to maintain hydration or rehydrate the skin. Moisturizers that prevent water from evaporating from the skin by providing a protective coating are called emollients. Additionally an emollient provides a softening or soothing effect on the skin surface and is generally considered safe for topical use. Preferred emollients include mineral oils, lanolin, petrolatum, capric/caprylic triglyceraldehydes, cholesterol, silicones such as dimeticone, cyclometicone, almond oil, jojoba oil, avocado oil, castor oil, sesame oil, sunflower oil, coconut oil and grape seed oil, cocoa butter, olive oil aloe extracts, fatty acids such as oleic and stearic, fatty alcohols such as cetyl and hexadecyl (ENJAY), diisopropyl adipate, hydroxybenzoate esters, benzoic acid esters of C₉₋₁₅-alcohols, isononyl iso-nonanoate, ethers such as polyoxypropylene butyl ethers and polyoxypropylene cetyl ethers, and C₁₂₋₁₅- alkyl benzoates, and mixtures thereof. The most preferred emollients are hydroxybenzoate esters, aloe vera, C₁₂₋₁₅-alkyl benzoates, and mixtures thereof. An emollient is present in an amount of about 1 wt.% to about 20 wt.% of the total weight of the composition. The preferred amount of emollient is about 2 wt.% to about 15 wt.%, and most preferably about 4 wt.% to about 10 wt.%.

Moisturizers that bind water, thereby retaining it on the skin surface are called humectants. Suitable humectants can be incorporated into a topical composition of the present invention such as glycerin, polypropylene glycol, polyethylene glycol, lactic acid, pyrrolidon carboxylic acid, urea, phopholipids, collagen, elastin, ceramides, lecithin sorbitol, PEG-4, and mixtures thereof. Additional suitable moisturizers are polymeric moisturizers of the family of water soluble and/ or swellable/ and/ or with water gelating polysaccharides such as hyaluronic acid, chitosan and/or a fucose rich polysaccharide which is e.g. available as Fucogel®1000 (CAS-Nr. 178463-23-5) by SOLABIA S. One or more humectants are optionally present at about 0.5 wt.% to about 8 wt.% in a composition of the present invention, preferably about 1 wt.% to about 5 wt.%.

The aqueous phase of the preferred topical compositions of the present invention can contain the usual cosmetic or pharmaceutical additives such as alcohols, especially lower alcohols, preferably ethanol and/ or isopropanol, low diols or polyols and their ethers, preferably propyleneglycol, glycerin, ethyleneglycol, ethyleneglycol monoethyl- or monobutylether, propyleneglycol monomethyl- or -monoethyl- or-monobutylether, diethyleneglycol monomethyl-or monoethylether and analogue products, polymers, foam stabilisators; electrolytes and especially one or more thickeners. Thickeners that may be used in formulations of the present invention to assist in making the consistency of a product suitable include carbomer, siliciumdioxide, magnesium and/ or aluminium silicates, beeswax, stearic acid, stearyl alcohol polysaccharides and their derivatives such as xanthan gum, hydroxypropyl cellulose, polyacrylamides, acrylate crosspolymers preferably a carbomer, such as carbopole^{®} of type 980, 981, 1382, 2984, 5984 alone or mixtures thereof. Suitable neutralizing agents which may be included in the composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginine and lysine and any combination of any foregoing. The neutralizing agent can be present in an amount of about 0.01 wt.% to about 8 wt.% in the composition of the present invention, preferably, 1 wt.% to about 5 wt.%.

The addition of electrolytes into the composition of the present invention may be necessary to change the behavior of a hydrophobic emulsifier. Thus, the emulsions/ microemulsions of this invention may contain preferably electrolytes of one or several salts including anions such as chloride, sulfates, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 wt.% to about 8 wt.% in the composition of the present invention.

The topical compositions of the invention can preferably be provided in the form of a lotion, a thickened lotion, a gel, a cream, a milk, an ointment, a powder or a solid tube stick and can be optionally be packaged as an aerosol and can be provided in the form of a mousse, foam or a spray. The compositions according to the invention can also be in the form of a suspension or dispersion in solvents or fatty substances, or alternatively in the form of an emulsion or microemulsion (in particular of O/W or W/O type, O/W/O or W/O/W-type), such as a cream or a milk, a vesicular dispersion, in the form of an ointment, a gel, a solid tube stick or an aerosol mousse. The emulsions can also contain anionic, nonionic, cationic or amphoteric surfactants.

The term "orally applicable composition" comprises any type of (fortified) food, (fortified) (animal) feed and beverages including also clinical nutrition, and also dietary supplements as well as the corresponding additives: food additives, beverage additives, feed additives. Also encompassed is functional food/functional feed i.e. a food/feed that has been enhanced with vitamins or pharmaceuticals to provide further specific health benefits, as well as a nutraceutical, i.e. a pill or other pharmaceutical product that has nutritional value.

The orally applicable compositions according to the present invention may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellyfying agents, gel forming agents, antioxidants and antimicrobials.

The compositions according to the present invention may further contain a compound selected from the group consisting of vitamin C and derivatives thereof (such ascorbylacetate, ascorbyltetraisopalmitate, Mg-ascorbylphosphate, Na-ascorbylphosphate, Na-ascorbylacetate), vitamin E and derivatives thereof (such as vitamin E-acetate), resveratrol, (-)-epigallocatechin gallate and mixtures thereof, preferably a compound selected from the group consisting of resveratrol, (-)-epigallocatechin gallate and mixtures thereof.

The orally applicable compositions according to the present invention may be in any galenic form that is suitable for oral administration to the animal body including the human body, e.g. in solid form such as (additives/supplements for) food or feed, food or feed premix, fortified food or feed, tablets, pills, granules, dragées, capsules, and effervescent formulations such as powders and tablets, or in liquid form such as solutions, emulsions or suspensions as e.g. beverages, pastes and oily suspensions. The pastes may be filled into hard or soft shell capsules, whereby the capsules feature e.g. a matrix of gelatin (from different origins like swine, cow or poultry), starch or starch derivatives or other polymers, e.g. cellulose derivatives. The orally applicable composition may be in the form of a controlled (delayed) release formulation.

Examples for fortified food include cereal bars, bakery items such as cakes and cookies.

Beverages encompass non-alcoholic and alcoholic drinks as well as liquid preparations to be added to drinking water and liquid food. Non-alcoholic drinks are e.g. soft drinks, sport drinks, fruit juices, lemonades, teas and milk based drinks. Liquid food are e.g. soups and dairy products.

In preferred embodiments of the present invention the orally applicable composition is a beverage containing lutein in an amount of 0.1 to 40 mg, preferably in an amount of 0.5 to 25 mg, and/or lycopene in an amount of 0.1 to 60 mg, preferably in an amount of 1 to 30 mg, and/or β-cryptoxanthin in an amount of 0.1 to 20 mg, preferably in an amount of 0.5 to 15 mg, and/or CoQ-10 in an amount of 1 to 500 mg, preferably in an amount of 5 to 60 mg, per Liter [L].

In more preferred embodiments of the present invention the orally applicable composition is a beverage further containing β-carotene in an amount of 1 to 50 mg, preferably in an amount of 2 to 10 mg, and/or vitamin C in an amount of 100 to 5000 mg, preferably in an amount of 200 to 1000 mg, and/or vitamin E in an amount of 15 to 2000 mg, preferably in an amount of 100 to 500 mg, and/or resveratrol in an amount of 5 to 100 mg, preferably in an amount of 10-50 mg, and/or (-)-epigallocatechin gallate in an amount of 10 to 1500 mg, preferably in an amount of 15-500 mg, per L.

An especially preferred beverage for the uses according to the present invention contains 120 mg of vitamin C per serving, 21 mg of vitamin E per serving, 2.4 mg of β-carotene per serving, 1 mg of lutein per serving, 0.2 mg of β-cryptoxanthin per serving, 4.8 mg of lycopene per serving, 20 mg of resveratrol per serving and 300 mg of (-)-epigallocatechin gallate per serving, whereby the serving size is 240 mL.

Animals in the context of the present invention may be mammals including humans, fish, and birds.

Preferred examples of mammals beside humans include dogs, cats, guinea pigs, (jack) rabbits, hares, ferrets, horses, and ruminants (cattle, sheep and goat). Preferred fish are aquarium fish (goldfish, koi), cage birds, e.g. canary.

Further objects of the present invention are the following methods:
- A method of maintaining the energy metabolism, the energy flow and/or the energy production of skin or in skin of animals including humans comprising the step of administering, preferably of administering orally, an effective amount of at least one compound selected from the group consisting of β-carotene, lutein, lycopene and β-cryptoxanthin, preferably consisting of lutein, lycopene and β-cryptoxanthin, and mixtures thereof and combinations thereof with CoQ-10, including all their stereoisomers, to said animal in need thereof.
- A method of maintaining the respiratory function of the skin of animals including humans comprising the step of administering, preferably of administering orally, an effective amount of at least one compound selected from the group consisting of β-carotene, lutein, lycopene and β-cryptoxanthin, preferably consisting of lutein, lycopene and β-cryptoxanthin, and mixtures thereof and combinations thereof with CoQ-10, including all their stereoisomers, to said animal in need thereof.
- A method of preventing UV-A radiation-induced mtDNA mutagenesis in skin of animals including humans comprising the step of administering, preferably of administering orally, an effective amount of at least one compound selected from the group consisting of β-carotene, lutein, lycopene and β-cryptoxanthin, preferably consisting of lutein, lycopene and β-cryptoxanthin, and mixtures thereof and combinations thereof with CoQ-10, including all their stereoisomers, to said animal in need thereof.

Effective amounts of lutein in these three methods are such amounts that the concentration of lutein in the blood plasma after administration ranges between 0.25 and 3 µM.

Effective amounts of lycopene in these three methods are such amounts that the concentration of lycopene in the blood plasma after administration ranges between 0.25 and 3.0 µM, preferably between 0.5 and 2.0 µM.

Effective amounts of β-cryptoxanthin in these three methods are such amounts that the concentration of β-cryptoxanthin in the blood plasma after administration ranges between 0.1 and 2 µM.

Effective amounts of CoQ-10 in these three methods are such amounts that the concentration of CoQ-10 in the blood plasma after administration ranges between 5 and 8 µM.

In preferred embodiments of these methods said compound(s)/carotenoid(s) is/are combined with at least one compound selected from the group consisting of vitamin C, vitamin E, resveratrol and (-)-epigallocatechin gallate, preferably with a compound selected from the group consisting of resveratrol and (-)-epigallocatechin gallate.

According to the present invention at least one compound selected from the group consisting of β-carotene, lutein, lycopene, and β-cryptoxanthin and mixtures thereof and combinations thereof with CoQ-10 can also be used in sunscreens as well as daily care products to improve their photoprotective potential, as well as effective micronutrient for skin maintenance, especially for protecting the energy metabolism of/the energy flow in/the energy production in and the respiratory function of skin of animals including humans.

Thus, also an object of the present invention is a method of improving the photoprotective potential of sunscreens and daily care products comprising the step of adding an effective amount of at least one compound selected from the group consisting of β-carotene, lutein, lycopene and β-cryptoxanthin and mixtures thereof and combinations thereof with CoQ-10 including all their stereoisomers. Here the concentrations of lutein may vary between 0.01 and 1000 ppm, more preferably between 0.1 and 50 ppm, and most preferably between 0.5 and 5 ppm, based on the total weight of the sunscreen or the daily care product. The concentrations of lycopene may vary between 0.01 and 1000 ppm, more preferably between 0.1 and 50 ppm, and most preferably between 0.5 and 10 ppm, based on the total weight of the sunscreen or the daily care product. The concentrations of CoQ-10 may vary between 0.001 and 1.0 weight-%, more preferably between 0.01 and 0.3 weight-%, based on the total weight of the sunscreen or the daily care product. The concentrations of β-cryptoxanthin may vary between 0.01 and 1000 ppm, more preferably between 0.1 and 10 ppm, and most preferably between 0.2 and 5 ppm, based on the total weight of the sunscreen or the daily care product.

Furthermore, according to the present invention at least one compound selected from the group consisting of lutein, lycopene, and β-cryptoxanthin, and mixtures thereof and combinations thereof with CoQ-10, can also be used in sunscreens as well as daily care products to improve their photoprotective potential, as well as effective micronutrient for skin maintenance, especially for protecting the energy metabolism of/the energy flow in/the energy production in and the respiratory function of skin of animals including humans.

Thus, also an object of the present invention is a method of improving the photoprotective potential of sunscreens and daily care products comprising the step of adding an effective amount of at least one carotenoid selected from the group consisting of lutein, lycopene, and β-cryptoxanthin or a mixture thereof or a combination thereof with CoQ-10. Here the concentrations of lutein may vary between 0.01 and 1000 ppm, more preferably between 0.1 and 50 ppm, and most preferably between 0.5 and 5 ppm, based on the total weight of the sunscreen or the daily care product. The concentrations of lycopene may vary between 0.01 and 1000 ppm, more preferably between 0.1 and 50 ppm, and most preferably between 0.5 and 10 ppm, based on the total weight of the sunscreen or the daily care product. The concentrations of β-cryptoxanthin may vary between 0.01 and 1000 ppm, more preferably between 0.1 and 10 ppm, and most preferably between 0.2 and 5 ppm, based on the total weight of the sunscreen or the daily care product.

All active ingredients, especially β-carotene, lutein, lycopene, β-cryptoxanthin and CoQ-10 used in connection with the present invention can be of natural origin, i.e. isolated from natural sources or can be chemically synthesized including biotechnological/genetic engineering methods.

Especially preferred is lycopene commercially available from DSM Nutritional Products Ltd. (Kaiseraugst, Switzerland) under the trade names redivivo 10% FS, redivivo (lycopene) 10% WS.

Especially preferred are β-carotene, lycopene, lutein, CoQ-10 and β-cryptoxanthin commercially available from DSM Nutritional Products Ltd. (Kaiseraugst, Switzerland). Product forms of lycopene, lutein and CoQ-10 are e.g. known under the trade names redivivo™ (lycopene) 10% FS, redivivo™ (lycopene) 10% WS, redivivo™ (lycopene) 5% TG or TG/P, Lutein CWS/S-TG, TG or TG/P, and All-Q™ (Coenzyme Q10) 10% TG/P or CWS/S from DSM Nutritional Products.

Other suitable commercially available products are LycoVit (Lycopene) TG 10%, Lycovit 20% Dispersion, Lutein 5% DC or Coenzyme Q10 10% DC from BASF, as well as Lyco-beads 5%, LycoPen 2% SG Dispersion and Lyc-O-Mato 15% from Lycored, FloraGlo (lutein) from Kemin, Xangold^{®} 10% Natural Lutein Esters Beadlet from Cognis, the following lutein forms from PIVEG: Lutein as Beadlets - 25%; Beadlets CWD 25% (Dispersible in cold water); Oil - 20%; Powder - 70%, and CoQ10 from Kaneka.

The invention is further illustrated by the following examples.

### Examples

### General

### Chemicals

The carotenoids lutein, lycopene, β-cryptoxanthin and zeaxanthin are from the chemical laboratories of DSM Nutritional Products AG (Kaiseraugst, Switzerland). Ammonium acetate p.a, butylated hydroxy toluene (BHT) p.a., tetrahydrofuran (THF) p.a., triethylamine p.a., are from Fluka Chemie AG (Buchs, Switzerland) and all other chemicals are from Merck (Darmstadt, Germany).

### Standard solutions

Stock solutions of lutein, lycopene, β-cryptoxanthin and zeaxanthin were prepared by dissolving 3-5 mg of the compound in a 0.025 % BHT (v/w) solution. Immediately afterwards, concentrations of suitable dilutions were determined by photometric measurements according to the Lambert-Beer law and based on the extinction (1%/1cm).

### Culture medium

Delivery of lutein, lycopene, β-cryptoxanthin and zeaxanthin to the cultured cells was carried out as described in Free Radicals Biol. Med. 2003, 34, 456-464. In brief, stock solutions described above with THF and 0.025% BHT as preservative as vehicles were used for delivering intact carotenoid and control solutions to cultured normal human fibroblasts. The concentration of the carotenoids applied was measured photometrically, as described above, and THF and the carotenoids in THF solution were used in the culture medium at the concentrations indicated ranging from 0 to 3µM.

### Cell culture

Skin fibroblast cultures were established from human foreskin samples at circumcision and cultured in Eagle's minimum essential medium (PAA Laboratories GmbH, Linz, Austria). The cells were grown to confluence in a humidified atmosphere containing 5 % CO₂.

### Example 1: UV-A-Irradiation of human dermal fibroblasts cells in the presence of lutein, lycopene, β-cryptoxanthin and zeaxanthin and analysis

### UV-A-Irradiation

Irradiation was carried out as described in J. Biol. Chem. 1999, 274, 15345-15349. The cells were irradiated with 8 J/cm² UV-A radiation from a Sellas Sunlight (Systems Dr. Sellmeier) irradiation device. The UV-A output was determined with a UV-A-Meter (Waldmann GmbH & Co. KG, Villingen-Schwenningen, Germany) and found to be approximately 70 milliwatts per squarecentimeter at a tube-to-target distance of 30 cm. To induce the common deletion, the cells were irradiated three times daily for 4 consecutive days during one or two consecutive weeks. The cells were then harvested by trypsinisation. One half of the cells was kept for DNA extraction and the other half was replated for ongoing culture.

### DNA Extraction

Total cellular DNA was extracted from normal human fibroblasts employing the QIAmp Tissue Kit (Qiagen, Hilden, Germany).

### PCR Analysis

Nested PCR was performed as described in J. Invest. Dermatol. 1998, 110, 149-152 and Leukemia 1995, 9, 1704-1710. Briefly a PCR product of 247 base pairs in length was amplified with primers C1 and C2 to be able to estimate the total amount of mtDNA in solution thus serving as a reference fragment. The fragments representing the common deletion were amplified by a combination of primer pair A1/A2 and B1/B2. Primer oligonucleotides A1/A2 were designed to anneal outside the region of the common deletion in the mitochondrial genome. In addition, the polymerase extension time was chosen to be too short for the complete amplification of wild-type PCR-products, resulting in the efficient amplification of only the shorter and deleted mtDNA-fragments. To increase sensitivity and specificity, a secondary nested PCR was performed (B1/B2) from the primary PCR-product. Linear amplification conditions for each primer pair up to 35 cycles were determined as described by P. Henninger et al. in Biol. Chem. Hoppe-Seyler 1993, 374, 625-629. Primary PCR (A1/A2 and C1/C2) was performed in 100 µl reaction volume with approximately 0.1 - 0.3 µg of genomic DNA and 0.5 units of Taq polymerase. The primer and nucleotides were used in concentration of 1 and 400 µM respectively. The PCR products were amplified in a Perkin-Elmer DNA Thermal Cycler 480 (Perkin-Elmer Applied Biosystems, Weiterstadt, Germany), primer oligonucleotides were synthesized by Invitrogen (Karlsruhe, Germany).

### Primer Oligonucleotides

The following sequences and nucleotide positions (shown in parentheses) are according to Anderson et al. in Nature 1981, 290, 457-465: A1, 5' GCA GTA ATA TTA ATA ATT TTC ATG 3' (7293-7316); A2, 5' CTA GGG TAG AAT CCG AGT ATG TTG 3' (13928-13905); B1, 5' TGA ACC TAC GAG TAC ACC GA 3' (7901-7920); B2, 5' GGG GAA GCG AGG TTG ACC TG 3' (13650-13631); C1, 5' ATG CTT GTA GGA CAT AAT AA 3' (219-238); C2, 5' AGT GGG AGG GGA AAA TAA TA 3' (466-447).

### DNA gel-electrophoresis

was performed in a 1 % agarose gel stained with ethidium bromide (0.20 µl/ml) (Sigma-Aldrich Chemie GmbH, Munich, Germany).

### Restriction Enzyme Analysis

To validate the identity of the amplified PCR products the fragments were subjected to diagnostic digestion with the restriction enzyme XbaI (New England Biolabs).

### RESULTS

### Effect of Lycopene

A total of 3 independent experiments have been conducted. In all 3 experiments, lycopene was consistently found to protect fibroblasts against the UV-A radiation-induced generation of the common deletion.

The results of a representative experiment are shown in table 1: After 1 week, UV-A radiation is capable of inducing the "common deletion" as a marker mutation for large scale DNA deletions in human dermal fibroblasts by a factor of 18. Addition of lycopene inhibits the UV-A radiation-induced formation of the common deletion at all doses tested (0.25 - 3 µM). Complete protection was observed at 0.5, 1 µM and 2 µM.

**Table 1: Effect of lycopene**

| UV-A radiation + given concentration of lycopene in the cell | Prevalence of mtDNA deletion relative to untreated cells |
|---|---|
| 0 | 18.13 |
| 0.25 µM | 2.90 |
| 0.5 µM | 1.22 |
| 1.0 µM | 1.53 |
| 2.0 µM | 1.53 |
| 3.0 µM | 2.52 |

### Effect of Lutein

A total of 3 independent experiments have been conducted. In all 3 experiments, lutein was found to protect human dermal fibroblasts against the UV-A radiation-induced generation of the common deletion.

The results of a representative experiment are shown in table 2: After 1 week of UV-A irradiation, induction of the common deletion can be observed by a factor of 6. This induction can be completely prevented by lutein at all tested concentrations (0.25-3 µM).

**Table 2: Effect of lutein**

| UV-A radiation + given concentration of lutein in the cell | Prevalence of mtDNA deletion relative to untreated cells |
|---|---|
| 0 | 8.39 |
| 0.25 µM | 0.98 |
| 0.5 µM | 0.27 |
| 1.0 µM | 0.71 |
| 2.0 µM | 0.37 |
| 3.0 µM | 0.59 |

### Effect of cryptoxanthin

A total of 3 experiments have been conducted, a representative experiment is shown in figure 3. After 2 weeks, UV-A radiation induces the common deletion by a factor of 4. This induction is abrogated by β-cryptoxanthin at all concentrations tested.

**Table 3: Effect of β-cryptoxanthin**

| UV-A radiation + given concentration of β-cryptoxanthin in the cell | Prevalence of mtDNA deletion relative to untreated cells |
|---|---|
| 0 | 3.93 |
| 0.1 µM | 0.80 |
| 0.25 µM | 0.64 |
| 0.5 µM | 0.25 |
| 1.0 µM | 0.62 |
| 2.0 µM | 0.25 |
| 3.0 µM | 1.03 |

### Effect of zeaxanthin

A total of 3 experiments have been conducted. Zeaxanthin showed no effect in any of the experiments.

### Example 2: Preparation of a beverage

(C)WS = (cold) water soluble, S = starch, TG = tablet grade, CC = crystal clear.

| **Recipe Juice Base** | **[g]** |
|---|---|
| Orange juice conc. low pulp 65°Brix | 594.0 |
| Carrot juice concentrate 70°Brix | 84.0 |
| Lemon juice clear 65°Brix | 39.0 |
| Orange flavour oil | 0.5 |
| Ethanol p.a. | 0.5 |
| Water deionized | 265.72 |
| Ascorbic acid | 7.14 |
| β-Carotene 10% CWS/S | 1.43 |
| Lutein 5% CWS/S-TG | 1.14 |
| β-Cryptoxanthin 5% CWS | 0.2 |
| Lycopene 10% CWS/S-TG | 2.86 |
| Vitamin E 15 %CC | 8.57 |
| Total | 1000.0 |

- Dissolve carotenoid and vitamin E product forms in part of the deionized water.
- Mix juice concentrates and add remaining water and pre-dissolved carotenoid and vitamin E product forms, stir gently.
- Add ascorbic acid to the mixture and stir until dissolved.
- Pre-blend orange oil and ethanol to a homogeneous oily component and add to the juice concentrates.
- Pre-emulsify with a rotor-stator homogenizer.
- Homogenize with a high pressure homogenizer P₁ 200 bar, P₂ 50 bar.
- Pasteurise the emulsion (72°C, 15 sec).
- Emulsion should rest at least one day before use, store in cool place.

| **Recipe beverage** | **[g]** |
|---|---|
| Sugar syrup 64°Brix | 156.2 |
| Sodium benzoate | 0.2 |
| Water deionized | 43.6 |
| Teavigo^{™} | 0.1 |
| Beverage Base | 70.0 |
| Tap water to | 1000.0 |

Teavigo is a trademark of DSM Nutritional Products Ltd., Kaiseraugst, Switzerland, for a (-)-epigallocatechin gallate (EGCG).
- Dissolve sodium benzoate in deionized water, add sugar syrup and (-)-epigallocatechin gallate under stirring.
- Add beverage base under stirring.
- Adjust the mixture with tap water to one litre of beverage.
- Fill in glass bottles and pasteurize (72°C, 15 sec.).

### Example 3: Preparation of a beverage

In an analogous manner to example 2 the juice base is prepared:

| **Juice Base** | **[g]** |
|---|---|
| Orange juice conc. low pulp 65°Brix | 594.0 |
| Carrot juice concentrate 70°Brix | 84.0 |
| Lemon juice clear 65°Brix | 39.0 |
| Orange flavour oil | 0.5 |
| Ethanol p.a. | 0.5 |
| Water deionized | 265.72 |
| Ascorbic acid | 7.14 |
| Lutein 5% CWS/S-TG | 1.14 |
| Vitamin E 15 %CC | 8.57 |
| Total | 1000.0 |

The juice base is processed to the final beverage according to the procedure mentioned under example 2.

| **Recipe beverage** | **[g]** |
|---|---|
| Sugar syrup 64°Brix | 156.2 |
| Sodium benzoate | 0.2 |
| Water deionized | 43.6 |
| Teavigo^{™} | 0.1 |
| Beverage Base | 70.0 |
| Tap water to | 1000.0 |

### Example 4: Preparation of a beverage

In an analogous manner to example 2 the juice base is prepared:

| **Juice Base** | **[g]** |
|---|---|
| Orange juice conc. low pulp 65°Brix | 594.0 |
| Carrot juice concentrate 70°Brix | 84.0 |
| Lemon juice clear 65°Brix | 39.0 |
| Orange flavour oil | 0.5 |
| Ethanol p.a. | 0.5 |
| Water deionized | 265.72 |
| Ascorbic acid | 7.14 |
| β-Cryptoxanthin 5% CWS | 0.2 |
| Vitamin E 15 %CC | 8.57 |
| Total | 1000.0 |

The juice base is processed to the final beverage according to the procedure mentioned under example 2.

| **Recipe beverage** | **[g]** |
|---|---|
| Sugar syrup 64°Brix | 156.2 |
| Sodium benzoate | 0.2 |
| Water deionized | 43.6 |
| Teavigo^{™} | 0.1 |
| Beverage Base | 70.0 |
| Tap water to | 1000.0 |

### Example 5: Preparation of a beverage

In an analogous manner to example 2 the juice base is prepared:

| **Juice Base - 4** | **[g]** |
|---|---|
| Orange juice conc. low pulp 65°Brix | 594.0 |
| Carrot juice concentrate 70°Brix | 84.0 |
| Lemon juice clear 65°Brix | 39.0 |
| Orange flavour oil | 0.5 |
| Ethanol p.a. | 0.5 |
| Water deionized | 265.72 |
| Ascorbic acid | 7.14 |
| Lycopene 10% CWS/S-TG | 2.86 |
| Vitamin E 15 %CC | 8.57 |
| Total | 1000.0 |

The juice base is processed to the final beverage according to the procedure mentioned under example 2.

| **Recipe beverage** | **[g]** |
|---|---|
| Sugar syrup 64°Brix | 156.2 |
| Sodium benzoate | 0.2 |
| Water deionized | 43.6 |
| Teavigo^{™} | 0.1 |
| Beverage Base | 70.0 |
| Tap water to | 1000.0 |

### Example 6: Preparation of a cereal bar

Redivivo is a trademark of DSM Nutritional Products Ltd., Kaiseraugst, Switzerland, for a lycopene.

| **Ingredients for 1 kg recipe** | **[g]** |
|---|---|
| Milled fructose | 120.0 |
| Honey | 150.0 |
| Crisp rice | 145.0 |
| Corn flakes | 100.0 |
| Raisins | 110.0 |
| Almonds | 85.0 |
| Coconut flakes | 90.0 |
| Vegetable fat | 60.0 |
| Emulsifier | 5.0 |
| Glucose syrup | 58.0 |
| Maltodextrin powder DE 10 | 71.0 |
| Dry mixed tocopherols 30% | 0.1 |
| Water | 9.0 |
| Redivivo® (Lycopene) 10% WS | 0.5 |
| or | |
| Lutein 5% CWS/S | 1.0 |

- Preblend Lycopene 10% WS or Lutein 5% CWS/S (commercially available from DSM Nutritional Products Ltd., Kaiseraugst, Switzerland) with the other dry ingredients, e.g. maltodextrin powder, milled fructose.
- Blend all ingredients in a planetary bowl mixer until a homogenous mixture is achieved.
- Spread the mixture on a greased baking tray to a thickness of approximately 1 cm.
- Bake at 180°C for 20 minutes.
- Let the cereal bar plate cool to ambient temperature and cut into bar-size pieces.
- Pack and seal in aluminium pouches.

### Example 7: Preparation of a CoQ-10 straight tablet

| | Composition | Label Claim | Overage | Quantities |
|---|---|---|---|---|
| | | Mg | % | Mg/Tabl. |
| | | | | |
| 1 | Coenzyme Q 10, directly compressible form | 30 | 20 | |
| | As ALL-Q 10% TG/P | | | 360.00 |
| 2 | Lacotse monohydrate, agglomerated | | | |
| | As Tablettose 80 (1) | | | 416.00 |
| 3 | Glyceryl behenate, atomized | | | |
| | As Compritol 888 ATO (2) | | | 16.00 |
| 4 | Crospovidone | | | |
| | As Polyplasdone XL-10 (3) | | | 8.00 |
| | Total Tablet Weight | | | 800.00 |

### Suppliers of excipients

(1) Tablettose 80: Fa. Meggle, Megglestr. 6-12, 83512 Wasserburg, Germany
(2) Compritol 888 ATO: Fa. Gattefosse AG, Haldenstr. 11, 6006 Luzern, Switzerland
(3) Polyplasdone XL 10, ISP Technologies Inc., Wayne, New Jersey, USA

### Procedure

I 1,2 and 4 are mixed in a tumbler mixer for 5 min
II 3 is added to I and mixed for 20 min
III Compress to tablets

### Example 8: Preparation of a lycopene, lutein, CoQ-10 and β-cryptoxanthin containing tablet

| | Composition | Label Claim | Overage | Quantities |
|---|---|---|---|---|
| | | Mg | % | Mg/Tabl. |
| | | | | |
| 1 | Coenzyme Q10, directly compressible form | 30 | 20 | |
| | As ALL-Q 10% TG/P | | | 360.00 |
| 2 | Lycopene, directly compressible form | 10 | 20 | |
| | As redivivo 5% TG/P | | | 240.00 |
| 3 | Lutein, directly compressible form | 5 | 20 | |
| | As Lutein 5% CWS/S-TG | | | 120.00 |
| 4 | Beta-Cryproxanthin, directly compressible form | 3 | 20 | |
| | As beta-Cryptoxanthin 5% beadlets | | | 72.00 |
| 5 | Lactose monohydrate, agglomerated | | | |
| | As Tablettose 80 (1) | | | 784.00 |
| 6 | Crospovidone | | | |
| | As Polyplasdone XL-10 (2) | | | 16.00 |
| 7 | Magnesium stearate | | | 8.00 |
| | Total Tablet Weight | | | 1600.00 |

### Suppliers of excipients

(1) Tablettose 80: Fa. Meggle, Megglestr. 6-12, 83512 Wasserburg, Germany
(2) Polyplasdone XL 10, ISP Technologies Inc., Wayne, New Jersey, USA

### Procedure

I 1 - 6 are mixed in a tumbler mixer for 15 minutes
II 7 is added to I and mixed for 2 minutes
III Compress to tablets

## Claims

1. Use of a compound selected from the group consisting of β-carotene, lutein, lycopene, β-cryptoxanthin, and mixtures thereof and combinations thereof with CoQ-10, for maintaining the energy metabolism, the energy flow and/or the energy production in skin or of skin of animals including humans.

2. Use of a compound selected from the group consisting of β-carotene, lutein, lycopene, β-cryptoxanthin, and mixtures thereof and combinations thereof with CoQ-10, for maintaining the respiratory function of the skin of animals including humans.

3. Use of a compound selected from the group consisting of β-carotene, lutein, lycopene and β-cryptoxanthin, and of mixtures thereof and combinations thereof with CoQ-10, for energizing the skin, maintaining and supporting the radiance and natural glow of the skin of animals including humans and for promoting a healthy appearance of the skin of animals including humans.

4. Use of a compound selected from the group consisting of β-carotene, lutein, lycopene, β-cryptoxanthin, and mixtures thereof and combinations thereof with CoQ-10, for preventing UV-A radiation-induced mtDNA mutagenesis in skin of animals including humans.

5. Use of a compound selected from the group consisting of β-carotene lutein, lycopene, β-cryptoxanthin, and mixtures thereof and combinations thereof with CoQ-10 for the manufacture of a composition, preferably an orally applicable composition, used for maintaining the energy metabolism, the energy flow and/or the energy production in skin or of skin of animals including humans, for maintaining the respiratory function of the skin of animals including humans, for energizing the skin, maintaining and supporting the radiance and natural glow of the skin of animals including humans and for promoting a healthy appearance of the skin of animals including humans and for preventing UV-A radiation-induced mtDNA mutagenesis in skin of animals including humans.

6. Use of a compound selected from the group consisting of β-carotene lutein, lycopene, β-cryptoxanthin, and mixtures thereof and combinations thereof with CoQ-10, for improving the photoprotective potential in sunscreens or daily care products.

7. Use of a compound selected from the group consisting of β-carotene, lutein, lycopene, β-cryptoxanthin, and mixtures thereof and combinations thereof with CoQ-10, as effective micronutrient for skin maintenance, especially for protecting the energy metabolism, the energy flow and/or the energy production and/or the respiratory function of skin of animals including humans.

8. The use according to any one of claims 1 to 7, wherein the compound or mixture is combined with at least one compound selected from the group consisting of β-carotene, vitamin C, vitamin E, resveratrol and (-)-epigallocatechin gallate, more preferably with at least one compound selected from the group consisting of vitamin C, vitamin E, resveratrol and/or (-)-epigallocatechin gallate, most preferably with resveratrol and/or (-)-epigallocatechin gallate.

9. Use of a compound selected form the group consisting of lutein, lycopene and β-cryptoxanthin and of mixtures thereof according to any one of claims 1-8.

10. A composition, preferably an orally applicable composition, comprising an effective amount of at least one compound selected from the group consisting of β-carotene, lutein, lycopene and β-cryptoxanthin and mixtures thereof and combinations thereof with CoQ-10 as active ingredient(s), **characterized in that** the amount is effective for maintaining the energy metabolism, the energy flow and/or the energy production in skin or of skin of animals including humans, for maintaining the respiratory function of the skin of animals including humans, for energizing the skin, maintaining and supporting the radiance and natural glow of the skin of animals including humans and for promoting a healthy appearance of the skin of animals including humans and for preventing UV-A radiation-induced mtDNA mutagenesis in skin of animals including humans.

11. A composition as in claim 10 comprising an effective amount of at least one compound from the group consisting of lutein, lycopene and β-cryptoxanthin as active ingredient.

12. The composition according to claim 10 or claim 11, **characterized in that** it is (fortified) food, a beverage, (fortified) feed or a corresponding additive, functional food, functional feed, a nutraceutical, a clinical nutrition or a dietary supplement.

13. The composition according to any one of claims 10-12, **characterized in that** it is in the form of a tablet, a pill, a granule, a dragée, a capsule or an effervescent formulation.

14. The composition according to any one of claims 10-13, **characterized in that** the daily amount of lutein ranges from 0.1 to 40 mg, and/or the daily amount of lycopene ranges from 0.1 to 60 mg, and/or the daily amount of β-cryptoxanthin ranges from 0.1 to 20 mg, and/or the daily amount of CoQ-10 ranges from 1 to 500 mg, for a human of 70 kg of weight.

15. The composition according to claim 10 or claim 11, **characterized in that** it is a beverage containing lutein in an amount of 0.2 to 50 mg, and/or lycopene in an amount of 2 to 50 mg, and/or β-cryptoxanthin in an amount of 0.1 to 30 mg, and/or CoQ-10 in an amount of 1 to 200 mg, per L.

16. The composition according to any one of claims 10-15, wherein said compound(s) is/are combined with at least one compound selected from the group consisting of β-carotene, vitamin C, vitamin E, resveratrol and (-)-epigallocatechin gallate, preferably with a compound selected from the group consisting of vitamin C, vitamin E, resveratrol and (-)-epigallocatechin gallate, preferably with resveratrol and/or (-)-epigallocatechin gallate.

17. Method of maintaining the energy metabolism, the energy flow and/or the energy production in skin or of skin of animals including humans comprising the step of administering, preferably of administering orally, an effective amount of at least one compound selected from the group consisting of β-carotene lutein, lycopene, and β-cryptoxanthin, and mixtures thereof and combinations thereof with CoQ-10, to said animal in need thereof.

18. Method of maintaining the respiratory function of the skin of animals including humans comprising the step of administering, preferably of administering orally, an effective amount of at least one compound selected from the group consisting of β-carotene, lutein, lycopene, and β-cryptoxanthin, and mixtures thereof and combinations thereof with CoQ-10, to said animal in need thereof.

19. Method of preventing UV-A radiation-induced mtDNA mutagenesis in skin of animals including humans comprising the step of administering, preferably of administering orally, an effective amount of at least one compound selected from the group consisting of β-carotene lutein, lycopene and β-cryptoxanthin, and mixtures thereof and combinations thereof with CoQ-10, to said animal in need thereof.

20. Method of enegizing the skin, maintaining and supporting the radiance and natural glow of the skin of animals including humans and of promoting a healthy appearance of the skin of animals including humans comprising the step of administering, preferably of administering orally, an effective amount of at least one compound from the group consisting of β-carotene, lutein, lycopine and β-cryptoxathin, and mixtures thereof, and combinations thereof with CoQ-10, to said animal in need thereof.

21. The method of any one of claims 17-20 wherein and effective amount of at least one compound selected from the group consisting of lutein, lycopene and β-cryptoxanthin is administered.

22. The method according to any one of claims 17-21, wherein said compound(s) is/are combined with at least one compound selected from the group consisting of β-carotene, vitamin C, vitamin E, resveratrol and (-)-epigallocatechin gallate, preferably from the group consisting of vitamin C, vitamin E, resveratrol and (-)-epigallocatechin gallate, more preferably with resveratrol and/or (-)-epigallocatechin gallate.

23. The method according to any one of claims 17 - 21, wherein the compound is lutein and its concentration in the blood plasma after administration ranges from 0.25 to 3 µM, and/or the compound is lycopene and its concentration in the blood plasma after administration ranges from 0.25 to 3.0 µM, preferably from 0.5 to 1.0 µM, and/or the compound is β-cryptoxanthin and its concentration in the blood plasma after administration ranges from 0.1 to 2 µM, preferably from 0.25 to 0.5 µM, and/or the compound is CoQ-10 and its concentration in the blood plasma after administration ranges from 5 to 10 µM, preferably from 5 to 8 µM.

24. Method of improving the photoprotective potential of sunscreens and daily care products comprising the step of adding an effective amount of at least one compound selected from the group consisting of β-carotene, lutein, lycopene, and β-cryptoxanthin, preferably from the group consisting of lutein, lycopene and β-cryptoxanthin, and mixtures thereof and combinations thereof with CoQ-10.
